# EUROPEAN PATENT APPLICATION

(11) **EP 4 725 434 A1**
(43) Date of publication of application: **15.04.2026**
(21) Application number: 24205741.2
(22) Date of filing: 10.10.2024
(51) Int. Cl.: A61B 18/18

(54) **SKIN TREATMENT DEVICE**

(71) Applicant: Koninklijke Philips N.V., 5656 AG Eindhoven (NL)
(72) Inventor: VAN ABEELEN, Frank Anton, 5656 AG Eindhoven (NL); BOURQUIN, Yannyk Parulian Julian, 5656 AG Eindhoven (NL); KRANS, Jan Martijn, 5656 AG Eindhoven (NL); SCHAEFERS, Klaus, 5656 AG Eindhoven (NL); LEMMENS, Paul Marcel Carl, 5656 AG Eindhoven (NL); CUBA GYLLENSTEN, Illapha Gustav Lars, 5656 AG Eindhoven (NL); BEREZHNOY, Igor, 5656 AG Eindhoven (NL); MENDOZA, Jose Luis Diaz, 5656 AG Eindhoven (NL); TAS, Sinem, 5656 AG Eindhoven (NL)
(74) Representative: Philips Intellectual Property & Standards

(57) **Abstract**

A method of generating guidance for a user of a personal care device with regards to at least one aspect of user-control of the device by means of a controlled acoustic output.

## Description

### FIELD OF THE INVENTION

The invention is related to the field of personal skin treatment devices.

### BACKGROUND OF THE INVENTION

Personal care devices can be operated by a user to perform a personal care function. The device is designed to be used in a certain way, in accordance with a set of best operating practices designed by the device manufacturer. These may include applying the device for a target duration period, moving the device in a certain way, and/or activating an operative function of the device at a certain rate or in a certain pattern. A user may not always understand how to operate the device in accordance with best practice.

One category of personal care device is that of personal skin care devices. The category of personal skin care devices includes, by way of non-limiting example: optical skin therapy devices, e.g. LED light therapy devices; exfoliation devices, facial cleaning devices, skin scrubber devices, RF skin tightening devices, and hair removal devices.

One example of a hair removal device is an intense pulsed light photoepilator device.

These devices use intense pulsed light (IPL) generated for example from a Xenon flash lamp. The generated light is absorbed by melanin in the hair and hair matrix. This results in generation of heat within the hair follicles, causing damage to the hair follicle which ultimately inhibits or delays future hair growth. The light is emitted in pulses, wherein each pulse is typically referred to as a 'flash'. For some devices, the timings of the flashes are automatically controlled, so that the device emits flashes of light in a particular pattern and at a particular frequency throughout operation and the user operates the device by moving the portion of the device carrying the light emitting window across the skin of the body part being treated to ensure application of light across the whole area and avoid over-exposure of any one or more individual areas.

For a given body part to be treated, it is possible to define an optimal treatment schedule of one or a plurality of treatment sessions, wherein each session is of a certain target duration.

For example, for an average woman, a single treatment session applied to two lower legs comprises around 500 flashes when using a light emitting window of window size approximately 4 cm². The time duration of the corresponding treatment session will depend upon the flash rate at which the light is emitted. For example, dependent on the flash rate, the treatment session might last between 7 and 20 minutes for a trained user. A long-lasting hair reduction result can be obtained if treatment sessions are repeated at intervals of 2 to 4 weeks.

The more closely that the operation of the device and scheduling of treatment sessions adheres to the optimal target, the more effective the results.

Users of IPL devices, particularly new users, may find it difficult to correctly operate the device in accordance with all aspects of best practice, and to adhere to the correct treatment schedule. For example, for new users, correctly administering the target number of flashes across the complete skin area of a certain body part may initially take a relatively long time duration, since the user is still learning how to use the device. Some users might lose concentration, or become bored or distracted, or otherwise fail to correctly apply the full number of flashes across the whole body part area. This may result in an incomplete treatment. Furthermore, a user may not realize that clear results visible to the naked eye can require several treatment sessions to appear. A user might therefore cease continuation of a certain treatment schedule due to incorrectly concluding that treatment is not working effectively. Similar challenges and problems arise also for other skin treatment devices, and thus are not limited to only IPL hair removal devices.

It would be of benefit to provide a user additional guidance during use of a personal care device as to the best practice for one or more characteristics of device operation.

### SUMMARY OF THE INVENTION

The invention is defined by the claims.

According to examples in accordance with an aspect of the invention, there is provided a skin treatment system. The skin treatment system comprises a user-operable skin treatment device for application to skin. The skin treatment device comprises a skin treatment sub-system for administering a skin treatment to skin over the course of a treatment session. One or more characteristics of the skin treatment are controllable by the user through user operation of the device. The system further comprises an acoustic output device for generating an audible acoustic output. The system further comprises a processing device for controlling the acoustic output of the acoustic output device. The processing device is configured to obtain an indication of a target characteristic of the user operation of the device to be performed in a treatment session. The processing device is further configured to control the acoustic output device to generate an acoustic output, and wherein at least one characteristic of the acoustic output is configured in dependence upon the target characteristic of the user operation of the device.

Thus, embodiments of the invention are based on the concept of generating acoustic guidance for a user of a personal skin care device which guides the user as to at least one element of best operating practice of the device. This helps the user achieve better skin treatment results using the device. As will become apparent from subsequent descriptions, the use of an acoustic output to provide guidance is particularly advantageous because information about device movement frequency, device movement rhythm or patterns and device usage time can all be encoded in the acoustic properties of an acoustic output through, for example, control of the rhythm, frequency or duration of the acoustic output. Thus it provides a technically advantageous medium for communicating guidance information in the relevant technical context of guiding a user in physically operating a skin treatment device in a manner that requires movement of the device over the skin and/or activation of the device functionality at a certain frequency and/or rhythm.

For example, in advantageous embodiments, the at least one characteristic of the acoustic output comprises at least one acoustic property of the acoustic output. The at least one acoustic property may include a duration of the acoustic output and/or a rhythm of the acoustic output. The rhythm of the acoustic output may comprise a rhythm frequency.

The target characteristic of the user operation of the device may for example include a target duration time for the treatment session, a target movement pattern of the skin treatment device (e.g. a target movement frequency), and/or a target activation frequency of the skin treatment subsystem of the skin treatment device.

The acoustic output provides guidance to the user as to one or more target characteristics of their usage of the device during a treatment session

In some embodiments, the acoustic output may include music. Additionally or alternatively, in some embodiments, the acoustic output may include spoken text.

As noted above, in some embodiments, the at least one characteristic of the acoustic output comprises at least one acoustic property of the acoustic output. For example, the at least one acoustic property may include a duration of the acoustic output, a rhythm or beat frequency of the acoustic output, a volume of the acoustic output, a tone of the acoustic output, and/or an audio frequency composition of the acoustic output. In other words, it is proposed to encode the guidance information in one or more acoustic or audible properties of the acoustic output. For example, a target movement frequency of the device by the user can be encoded in the rhythm frequency of the device, or a target duration of the treatment session can be encoded in the duration of the acoustic output.

In some embodiments, the generating of the acoustic output comprises retrieving an audio file and controlling the acoustic output device to generate an acoustic output based on the audio file. In other embodiments, the generating of the acoustic output may comprise composing the acoustic output on the fly using a sound generating module.

According to at least one set of embodiments, the target characteristic of the user operation of the device includes a target duration time of the treatment session. The at least one characteristic of the acoustic output may include a duration time of the acoustic output, and the acoustic output is controlled such that a duration time of the generated acoustic output is no longer than the target duration time for the treatment session. In other words, the acoustic output is controlled so as to terminate or end or silence if and when the target duration time of the treatment session is reached during the treatment session.

The processing device may, in accordance with some examples within this set of embodiments, be configured to: detect a start time of a treatment session; obtain an indication of the target duration time for the treatment session; and generate a control signal to trigger a start of generation of the acoustic output at the detected start of the treatment session. The acoustic output may be controlled such that a duration time of the generated acoustic output is no longer than the target duration time for the treatment session.

According to this set of embodiments, the target characteristic or attribute of the user operation which is intended to be influenced is the operating time for which the user applies the skin treatment, i.e. the duration of the treatment session. The device is configured such as to permit the user to control the length of the treatment session, for example by permitting the user direct control of the activation and deactivation of the skin treatment subsystem, and/or by controlling application of the operative part of the skin treatment subsystem to the skin, e.g. by applying or removing the device from the skin surface. To guide the user as to an optimum duration for the skin treatment session, the acoustic output is controlled so as to have a maximum duration which does not exceed the target treatment session duration. In other words, in this set of embodiments, if the target duration time is reached while the treatment session is still ongoing, the acoustic output is controlled or configured to cease or stop, to thereby prompt the user that the target treatment duration has been completed and it is time to bring treatment to an end.

If the user stops use of the device (i.e. pauses or ends the treatment session) before the target duration time for the treatment session has been reached, there are different options for how the system may be configured to respond. According to one set of examples, the acoustic output is controlled so that the generation of the acoustic output ceases (the sound is quieted) when the user ceases to apply the skin treatment. Alternatively, according to a further set of examples, the generation of the acoustic output may continue in the event that the user ceases application of the skin treatment before the target duration time for the treatment session has been reached. It may be controlled to continue until the target duration time for the treatment session has been fully reached, or may be controlled to continue for a pre-determined period of time following cessation of application of treatment by the user, provided that the target duration time for the treatment session is not reached in the meantime.

In some examples, a user interface may be provided which permits a user to manually suppress the generation of the acoustic output, i.e. the user is provided an option to force the sound to end (e.g. stop button in a connected app).

In some embodiments, the obtaining of the indication of the target duration time for the treatment session may comprise: obtaining an indication of a target body part to be treated; and querying a treatment time database storing estimated treatment times for different body parts in accordance with the target body part to be treated. The processing device may determine a target duration time for the treatment session to be equal to the estimated treatment time for the target body part recorded in the treatment time database.

Thus, here, a standardized database or lookup table is used to store generic estimates of optimum duration times for treatment sessions for a given body part.

In some examples, the estimated treatment time for a given body part corresponds to an estimated time to execute a pre-defined number of treatment events using the skin treatment subsystem. In some embodiments, the processing device may be further configured to update the estimated treatment time recorded in the treatment time database for the target body part in dependence upon an actual time taken by a user to perform the pre-defined number of treatment events during an actual treatment session.

Thus, here, actual usage information is used to improve estimates of optimum treatment time.

The detecting of the start of a treatment session can be done in different ways.

In some embodiments, the skin treatment device comprises a motion sensor integrated therein and the processing device is configured to detect a start of a treatment session by detecting movement of the skin treatment device by a user using the motion sensor integrated in the skin treatment device. The processing device may be configured to detect occurrence of a pre-defined patten of motion for example.

In some embodiments, the processing device is configured to detect a start of a treatment session by detecting operation by the user of a user control element comprised by the skin treatment device, for example the pressing of a button by the user.

In some embodiments, the processing device is configured to detect a start of a treatment session by a manual indication of an intended start of a treatment session by a user by means of a user interface operatively coupled with the processing device, for example by means of a software application installed on a mobile computing device which is operatively coupled with the processing device.

In some embodiments, the target characteristic of the user operation of the device includes a target movement pattern of the device by the user and/or a target activation frequency of the skin treatment sub-system by the user. The target movement pattern may include a target movement frequency of the device.

Within this set of embodiments, in some examples, the at least characteristic of the acoustic output which is configured is a rhythm of the acoustic output. The processing device may be configured to generate the acoustic output such that the rhythm of the acoustic output corresponds rhythmically to the target movement pattern and/or target activation frequency.

For example, the rhythm of the acoustic output may have an associated rhythm frequency. In accordance with some embodiments, the processing device may be configured to generate the acoustic output such that the rhythm frequency of the acoustic output matches or substantially the target movement pattern (e.g. target movement frequency) or the target activation frequency.

The target movement frequency may be an intended frequency at which to move the skin treatment device from one skin location to another. The target activation frequency may be a target frequency of initiation of an instance of a skin treatment event or action by the skin treatment subsystem, e.g. activation of a light pulse in the case of a photo-epilation device. The latter is known as the flash rate.

Thus, here, the acoustic output provides guidance to the user as to a frequency or rate at which to activate a treatment operation of the device or at which to move the device. Instructions could be given to the user in advance telling them how they should use the frequency guidance provided by the acoustic output.

As noted above, in some embodiments, the generating of the acoustic output comprises retrieving one or more audio files from an audio file database and controlling the acoustic output device to generate an acoustic output based on the one or more audio files.

For example, in some embodiments, the retrieving of the audio file comprises communicating with an audio file database storing a plurality of audio files encoding different respective acoustic outputs differing from one another with regards to at least one acoustic property, e.g. duration and/or rhythm. The processing device may be configured to determine a target value of the at least one acoustic property for the acoustic output based on the target characteristic of user operation of the device. The processing device may be configured to select one or more of the plurality of audio files in accordance with the target value of the at least one acoustic property.

Thus here, audio files are used which record pre-determined acoustic outputs, and wherein one or more of the audio files is selected which encodes an acoustic output whose acoustic properties best match those which are required to provide the guidance to the user.

In some examples, the retrieving of the audio file comprises retrieving an audio file which encodes an acoustic output with an associated rhythm which corresponds rhythmically to a target motion pattern and/or activation frequency of the device.

In some embodiments, the audio file rhythm or frequency may be adjusted to match the target frequency of the characteristic of user operation of the device.

Additionally or alternatively, in some examples, the retrieving of the audio file comprises retrieving an audio file which encodes an acoustic output having a duration which matches or substantially matches a target duration time of the treatment session.

In some embodiments, the generating of the acoustic output comprises: retrieving an audio file; retrieving an indication of a pre-defined target movement pattern of the device and/or target activation frequency of the skin treatment sub-system by the user; and applying a modification to the pre-defined target movement pattern of the device and/or target activation frequency of the skin treatment sub-system by the user so as to improve a match between the rhythm frequency of the acoustic output encoded on the audio file and the target movement pattern of the device and/or target activation frequency of the skin treatment sub-system by the user.

Thus here, the target frequency of the characteristic of user operation of the device , e.g. a flash rate, is adjusted slightly to match the audio file rhythm or frequency.

In some embodiments, the retrieving of the audio file comprises the processing device retrieving and applying an AI selection algorithm for selecting the audio file based on the at least one acoustic property.

Instead of using pre-determined acoustic outputs, the acoustic output can be at least partially created in real time. One way to do this is using MIDI files which permit modification of one or more acoustic properties.

For example, in some embodiments, the retrieved audio file is a MIDI audio file, the MIDI audio file encoding an acoustic output having digitally encoded values for one or more acoustic properties. The processing device may be configured to determine a target value of at least one acoustic property for the acoustic output based on the target characteristic of user operation of the device. The processing device may be configured to control the acoustic output to have the target value for the at least one acoustic property by modifying the digitally encoded value of said at least one acoustic property encoded in the MIDI file.

In some embodiments, wherein the target characteristic of the user operation of the device includes a target movement pattern of the device by the user and/or a target activation frequency of the skin treatment sub-system by the user. The processing device may be configured to control the acoustic output device to generate an acoustic sound responsive to each movement of the device by the user and/or responsive to each activation of the skin treatment sub-system by the user, and wherein the acoustic sound is superposed atop the acoustic output associated with the audio file.

This provides interactive guidance to a user to guide them in controlling motion or activation of the device at the target frequency. If the audio file plays a musical output, the user will be able to tell if they are activating the device at the correct target frequency since the series of superposed sounds generated responsive to their pattern of activations will synchronize harmoniously with the rhythm frequency of the musical output. This provides a novel and effective means of guiding the user.

By way of example, in some embodiments, the skin treatment device may comprise a user control element permitting a user to activate the skin treatment subsystem by triggering instances of a treatment function of the device using the skin treatment sub-system. In some embodiments, the processing device is configured to control the acoustic output device to generate an acoustic sound responsive to each activation of the user control element by the user. The acoustic sound may be superposed atop the acoustic output associated with the audio file.

By way of further example, additionally or alternatively in some embodiments, the skin treatment device comprises a motion sensor for sensing user movement of the device and the processing device may be configured to control the acoustic output device to generate an acoustic sound responsive to each user movement of the device sensed by the movement sensor. The acoustic sound is superposed atop the acoustic output associated with the audio file.

In some embodiments, the acoustic output is a musical output, and wherein the acoustic sound generated responsive to each user activation of the user control element is a musical instrument sound.

In some embodiments, the skin treatment device may be a photo-epilation device, for example an Intense Pulsed Light (IPL) photoepilation device. However, the principles of the invention can be advantageously applied also to other types of personal care device, for example other types of skin treatment device.

In some embodiments, the acoustic output comprises music or spoken text.

Another aspect of the invention is a method for providing interactive guidance to a user during a treatment session using a user-operable skin treatment device to administer a skin treatment, wherein one or more characteristics of the skin treatment are controllable by the user through user operation of the device.

The method comprises: obtaining an indication of a target characteristic of the user operation of the device to be performed in a treatment session; controlling an acoustic output device to generate an acoustic output, and wherein at least one characteristic of the acoustic output is configured in dependence upon the target characteristic of the user operation of the device.

In some embodiments, the skin treatment device is a personal care device, for example a grooming device, for example a hair removal device, for example a photo-epilator device.

Another aspect of the invention is a computer program product comprising computer program code configured, when run on a processor, to cause the processor to perform a method as outlined above, or in accordance with any embodiment detailed in this disclosure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and method in accordance with one or more embodiments of the invention;
Fig. 3 is a block diagram schematically illustrating the processing flow in accordance with one or more embodiments of the invention;
Fig. 4 schematically illustrates an example Intense Pulsed Light (IPL) skin photo-epilation device; and
Fig. 5 schematically illustrates an example series of positions of an example IPL device on the skin for successive treatment events.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method of generating guidance for a user of a personal care device with regards to at least one aspect of user-control of the device by means of a controlled acoustic output.

There are different ways to encode guidance information in an acoustic output so as to be interpretable to a user listening to the acoustic output. Advantageously, embodiments of the invention propose to encode the information in one or more intrinsic acoustic properties of the acoustic output, such as duration, volume, tone, beat frequency, audio frequency composition and so on. It is considered that this is advantageous compared to encoding the information semantically via spoken instructions since (i) once the user has learned the association between the variable acoustic properties and the intended change in device operation it may be more quickly and directly interpretable by a user and (ii) this approach allows for easier adaptation of the guidance information according to variable characteristics of the treatment, e.g. session length, light pulse frequency and so on, and wherein this adaptation might be done in real time / on-the-fly / in situ by adaptation of the acoustic properties of the acoustic output.

By way of example, in accordance with at least one set of examples, an acoustic output in the form of music and/or spoken text is generated to stimulate the engagement of users of a photoepilation device, or more generally, of skin treatment devices and/or to guide the user to operate the device in accordance with a pre-defined set of standards.

In accordance with one set of examples, the proposed method may comprise one or more of the following steps:
- providing a skin treatment device for applying a treatment to the skin of a user; and
- playing a music/audio file to the user in synchronization with application of the treatment by the skin treatment device.

With regards to the meaning of "in synchronization", different options are possible, and various embodiments will be detailed in the forthcoming description.

According to some examples, the acoustic output may be generated by the retrieving and playing of an audio file. In some examples, the acoustic output may be generated by the skin treatment device itself. In other examples, an audio file may be retrieved and played by an app running on a mobile computing device such as a smartphone or tablet computer which may be communicatively connected (e.g. via a wireless connection such as Bluetooth or Wi-Fi) to the skin treatment device. The personal computing device may use an acoustic output device integral to the personal computing device to generate the acoustic output or the personal computing device may be communicatively coupled with a separate device integrating an acoustic output device, for example auxiliary speakers or headphones.

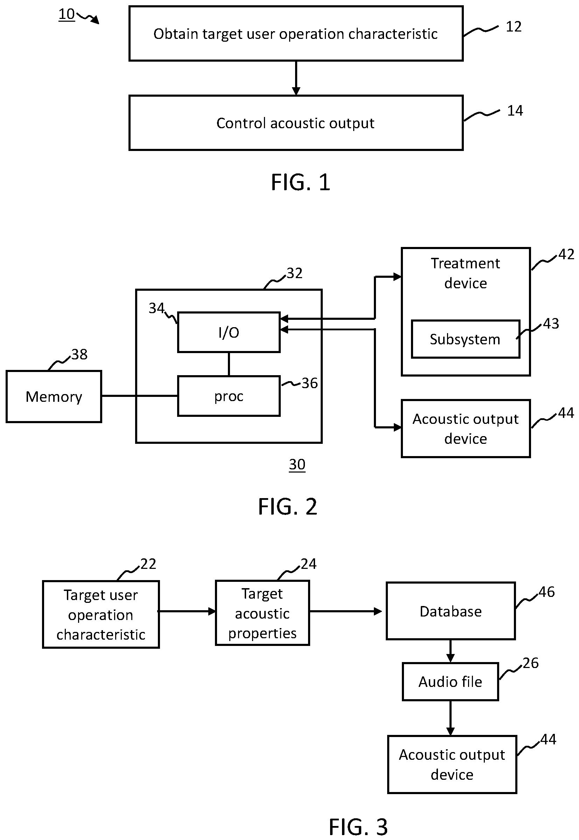

Fig. 1 outlines in block diagram form the basic steps of an example method according to one or more embodiments. The steps will be recited in summary, before being explained further in the form of example embodiments.

The provided method 10 is a method for providing guidance to a user during a treatment session using a user-operable skin treatment device to administer a skin treatment. The device is configured such that one or more characteristics of the skin treatment are controllable by the user through user operation of the device.

The method comprises obtaining 12 an indication of a target characteristic of the user operation of the device to be performed in a treatment session.

The method further comprises controlling 14 an acoustic output device to generate an acoustic output, wherein at least one characteristic of the acoustic output is configured in dependence upon the target characteristic of the user operation of the device.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The system may be referred to as a skin treatment system 30.

The processing device 32 alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises the skin treatment device 42. The skin treatment device comprises a skin treatment sub-system 43 for administering a skin treatment to skin over the course of a treatment session, and wherein one or more characteristics of the skin treatment are controllable by the user through user operation of the device. By way of non-limiting example, the skin treatment device may comprise an Intense Pulsed Light (IPL) device, such as an IPL photo-epilator device, and wherein the photo-epilator device comprises a photoepilation subsystem comprising e.g. a light source for generating treatment light for administration to hair follicles.

It is noted that a photo-epilator device is just one example skin treatment device to which embodiments of the invention might be applied. The principles of the invention can be advantageously applied also to other types of personal care device, for example other types of skin treatment device.

In the illustrated example of Fig. 2, the system further comprises the acoustic output device 44 for generating an audible acoustic output. The acoustic output device may for example comprise a speaker. The acoustic output device 44 may be integrated in the skin treatment device 42, for example a built-in speaker comprised by the skin treatment device. However, in other examples, the acoustic output device may be integrated in a separate device such as for example a mobile computing device with which the skin treatment device 42 is communicatively coupled, or a dedicated audio output device such as a set of headphones or an auxiliary speaker.

Furthermore, the processing device 32 may be integrated in the skin treatment device 42 in some examples, or it may be separate to the skin treatment device. In some examples, it may be a processing device comprised by a mobile computing device such as a smartphone or tablet computer.

In some embodiments, the system may be associated with a mobile computing device (not shown) which may be communicatively coupled with one or both of the skin treatment device 42 and the processing device 32. The mobile computing device may be provided as part of the system, or one or more components of the system may be arranged for communicating with the mobile computing device as an external component. As mentioned above, the mobile computing device may provide one or more of the integral components of the system, for example in the case where the acoustic output device 44 and/or the processing device 32 are integral components of the mobile computing device.

Additionally or alternatively, the mobile computing device may provide one or more user interface functionalities. For example, the mobile computing device may include a software application installed thereon, wherein the software application may permit a user to configure one or more settings or parameters of the skin treatment performed by the skin treatment device in a given treatment session.

The software application may further permit a user to input an indication of a target body part to be treated in advance of starting a treatment session for treating that body part. The software application may communicate with the skin treatment device 42 and/or the processing device 32 to configure parameters of the skin treatment in dependence upon the user-indicated target body part to be treated.

The software application may additionally or alternatively permit a user to trigger a start of a treatment session by means of a user input to the software application provided via the user interface of the mobile computing device.

The software application may additionally or alternatively provide a user output function, e.g. providing feedback information to a user regarding the treatment session, e.g. indicating an elapsed time of the treatment session or one or more other parameters of the treatment session.

The software application may additionally or alternatively provide functionality to permit a user to keep track of a treatment schedule comprised of multiple treatment sessions. For example, each treatment session may be recorded in a data log personalized to the user and enable the user to review timings and durations of past treatment sessions. The software application may guide or prompt the user as to the best next time for a next treatment session based on the body part being treated and based on the timings of past treatment sessions. The software application may generate push notifications to this effect in some embodiments.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim. The memory may be integrated in the skin treatment device 42 in some examples, or may be integrated in a separate device, such as a personal computing device. In some examples, the memory may be a cloud memory or other remote memory store accessible by the processing device via an internet or network link.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

With regards to the skin treatment, the skin treatment device 42 may be configured to apply skin treatment which is composed of a sequence of skin treatment actions or events or operations. For example, in the case of an Intense Pulsed Light (IPL) photoepilator device, a skin treatment event might comprise generation of a light pulse using a flash lamp. The skin treatment device 42 may in some embodiments be configured to automatically trigger skin treatment events at regular time intervals. The skin treatment events may for example be triggered at a defined skin treatment event frequency. The treatment device may additionally or alternatively permit a user to trigger instances of skin treatment events manually, e.g. by operating a user control element comprised by the device, e.g. by pressing a button.

As noted above, the system 30 may include a user interface. This may in some examples be at least partially facilitated by e.g. a mobile computing device. The user interface may permit a user to indicate a target body part to be treated ahead of a current treatment session. The user interface may permit a user to configure one or more settings or parameters of the skin treatment to be applied during a treatment session. The user interface may permit manual triggering by a user of a start of a treatment session.

In some embodiments, the skin treatment device 42 comprises one or more user-operable user control elements, for example buttons which the user can press. The user control elements may be operable by the user for controlling one or more elements of skin treatment device functionality. At least one user control element may be provided which permits a user to trigger instances of skin treatment events or actions by the skin treatment subsystem, e.g. pulses of light by an IPL lamp.

Upon starting a treatment session, the processing device 32 may configure settings or parameters of the skin treatment to be applied by the skin treatment device 42. The processing device may configure a frequency of automatic triggering of skin treatment events in some cases.

During a skin treatment session, the user operates the skin treatment device 42 to apply skin treatment to a target body part to be treated. The processing device 32 may be configured to track an elapsed time of a current treatment session. The processing device 32 may be configured to track a total number of skin treatment events or actions administered by the skin treatment subsystem, e.g. a total number of IPL light pulses. If more than one type of skin treatment event is possible, the processing device may track the total number of each type of skin treatment event.

With regards to the configuring of the acoustic output, in general terms, this can be done in at least two different ways.

One way is to communicate with an audio file database storing a plurality of audio files, each encoding a respective acoustic output, wherein the acoustic outputs encoded by the different audio files vary with regards to one or more acoustic properties. This approach is schematically illustrated in Fig. 3. The processing device may determine in advance required target acoustic properties 24 for the acoustic output in dependence upon the target characteristic of user operation 22. The processing device may communicate with an audio file database 46 storing the plurality of audio files. The processing device may then select an audio file 26 from the database encoding an acoustic output with acoustic properties which match the required target acoustic properties 24. The acoustic output device 44 may be controlled to generate an acoustic output based on the acoustic output encoded in the audio file 26.

Another way is to at least partially form or create the acoustic output in real time.

One approach is to use MIDI files. The processing device 32 may be configured to communicate with an audio file database storing a plurality of audio files, wherein at least a subset of the audio files are MIDI audio files encoding an acoustic output having configurable digitally encoded values for one or more acoustic properties. The processing device may retrieve a MIDI audio file. The processing device may determine required target acoustic properties for the acoustic output in dependence upon the target characteristic of user operation. The processing device may configure one or more of the configurable digitally acoustic properties of the acoustic output encoded by the MIDI audio file, e.g. beat frequency, tone, volume, duration.

Another approach is to use generative AI. The processing device 32 may be configured to determine required target acoustic properties for the acoustic output in dependence upon the target characteristic of user operation, and to generate an acoustic output with the target acoustic properties using a generative AI model.

As noted previously, in some embodiments, the target characteristic of the user operation of the device includes a target duration time of a treatment session.

Target duration time of the treatment session may be stored as a data item in a data store, e.g. a register or memory. The value of this data item may be set at manufacture or may be set during operation. The target duration time may in some embodiments be determined in dependence upon a target body part to be treated. For example, the processing device 32 may be communicable with a treatment time database storing estimated treatment times for different body parts, and the processing device may query the treatment time database according to a target body part to be treated to thereby determine a target duration time for the treatment session.

As noted above, in some embodiments, the target characteristic of the user operation of the device includes a target movement pattern of the device by the user and/or a target activation frequency of the skin treatment sub-system by the user. The target movement pattern may include a target movement frequency of the device. The target movement pattern and/or activation frequency may be stored as a data item in a data store, e.g. a register or memory. The value of this data item may be set at manufacture or may be set during operation. In some embodiments, the skin treatment subsystem may be operated by the device with an automated activation pattern, to result in an automatically triggered pattern of skin treatment events. The target movement pattern may be set so as to match the automated activation pattern of skin treatment subsystem. This way, the user is guided to move the device in synchrony with the automated activations of the skin treatment subsystem. For example, the skin treatment subsystem may be operated by the device with an automated activation frequency. For example, for an IPL device, this may correspond to a flash rate of the IPL lamp. The target movement pattern may be set so as to match the automated activation frequency of skin treatment subsystem.

Embodiments of the invention are for implementation in association with a personal care device, for example a personal skin treatment device. By way of example, embodiments of the invention may be implemented in associated with an Intense Pulsed Light (IPL) photoepilation device.

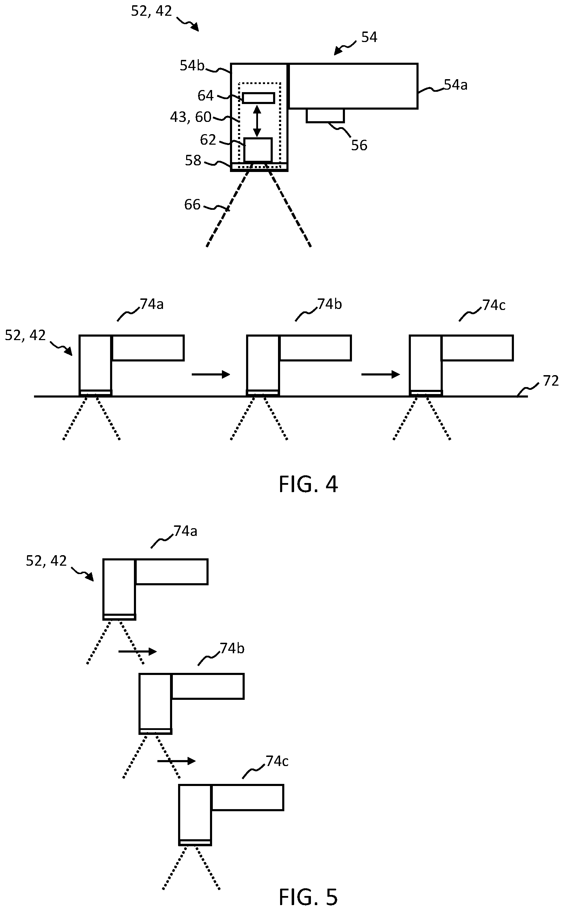

A schematic illustration of an example IPL photoepilation device 52 is shown in Fig. 4.

The IPL photoepilation device 52 comprises a housing 54, wherein the housing comprises a handle portion 54a for being held by a user during use and a head portion 54b. The head portion houses a skin treatment subsystem 43 in the form of an IPL light generation subsystem 60. The IPL light generation subsystem 60 comprises an IPL light source 62 and a local IPL controller 64 operatively coupled to the light source 62 for controlling the light source to generate a treatment light output 66. The light output 66 of the light source is emitted from the head portion 54b of the housing of the device via a light output window 58. The photoepilation device 52 may further comprise a user control element 56, such a button, which can be actuated or otherwise stimulated by the hand of the user to control an aspect of device operation, e.g. trigger emission of a light pulse from the light source. The emission of a light pulse may represent a treatment event in accordance with the language previously used in this disclosure.

During operation, the user holds the device 52 via the handle portion 54a of the housing of the device and can move the positioning of the device relative to the skin of the user. For example, Fig. 4 shows how a user might move the device between three example positions across the skin 72 of a user: a first position 74a, a second position 74b and a third position 74c. In each position, the user applies the head portion 64b of the device housing to the skin, with the light output window 58 pressed against the skin such that the treatment light output 66 is transmitted into the tissue of the user beneath the skin 72.

For ease of illustration, in Fig. 4, the three positions 74a, 74b, 74c are shown as quite far apart from one another. However, this is not to scale. In reality, the successive light pulses at successive positions may be approximately adjacent to one another. By way of example, Fig. 5 illustrates a further schematic representation (also not to scale) which better represents the proximity of successive positionings of the device for successive treatment events. For example, the light output projections of successive treatment events at successive positions may overlap spatially.

With regards to the light source 62, by way of example, the light source may be operable to generate a light output 66 having an optical wavelength the range of 500 to 1200 nanometers. This range is optimal for targeting melanin in the hair follicles while minimizing damage to the surrounding skin. The light source may comprise a xenon flash lamp. The intensity of the light output may be controllable.

With regards to the local controller 64, this may be configured to control the light source in accordance with a pre-defined treatment pattern or program, for example controlling emission of pulses of light at a controlled frequency and for a controlled duration. The controller 64 may control intensity of the light output 66 of each pulse.

The controller 64 may further be operatively coupled with the user control element 56 and configured to control operation of the light source 62 at least partially in dependence upon or responsive to user actuation of the user control element. In some embodiments, the user actuation of the control element 56, e.g. button, may trigger the controller to activate pulsing or flashing of the light source 62 at a regular pulse frequency. Each pulse may be referred to as a treatment event. In other words, the user can switch on or off automated emission of pulsed light, where the pulse frequency is controlled by the controller 64, for example in accordance with a pre-defined standard or protocol, and/or in accordance with defined user settings, e.g. defined via another user interface element, or via a smartphone app linked with the device. In further examples, the controller may be configured such that the user actuation of the control element 56 triggers emission of a single pulse, such that the user controls generation of individual pulses via actuation of the user control element 56. These pulses may be referred to as treatment events. In some examples, the controller 64 may be operable in each of these two different modes and can be switched between them: regular pulsing mode, vs user-controlled single pulse mode.

In either case, the user has some control over operation of the device. In the case where the local controller 64 controls the light source to flash at a regular pulse frequency, the user controls placement of the device on the body and controls movement of the device between different positions and the timings with which this movement occurs. In the case where the user triggers generation of light pulses using the user control element 56, the user still controls the movement of the device but in addition controls the timings of the light pulse generation. In both cases, the user also has control over the duration for which the device is used in a given treatment session.

Optionally, the system 30 may permit the user to configure one or more settings of the device operation in advance of use or during use. The configuration of the settings may be enabled by means of a user interface integrated in the skin treatment device 42 itself or a user interface which is separate to the skin treatment device. The configuration of the settings may be enabled by means of a software application installed on a mobile computing device such as a smartphone or tablet.

Embodiments of the invention provide guidance to a user by means of audible acoustic outputs for guiding the user in the operation of the device, in particular for guiding the user with regards to at least one target characteristic of user operation of the device.

According to one set of embodiments, the guidance information may relate to a duration time for which to apply treatment using the device to a particular body part, in other words a target duration time of the treatment session. In other words, the at least one target characteristic of user operation of the device includes a target duration time of a treatment session. This information may be encoded in the acoustic output via configuration of a duration time or a maximum duration time of the acoustic output.

For example, to guide the user as to an optimum duration for the skin treatment session, the acoustic output is controlled so as to have a maximum duration which does not exceed the target treatment session duration. In other words, in this set of embodiments, if the target duration time is reached while the treatment session is still ongoing, the acoustic output is controlled or configured to cease or stop, to thereby prompt the user that the target treatment duration has been completed and it is time to bring treatment to an end.

If the user stops use of the device (i.e. pauses or ends the treatment session) before the target duration time for the treatment session has been reached, there are different options for how the system may be configured to respond. According to one set of examples, the acoustic output is controlled so that the generation of the acoustic output ceases (the sound is quieted) when the user ceases to apply the skin treatment. This sudden interruption of the sound encourages the user to continue with the treatment until the natural end of the sound is reached, e.g. the end of the song or other musical piece or the end of the spoken output. Generation of the acoustic output may be re-continued responsive to the user restarting application of the skin treatment. When a total cumulative operation time of the skin treatment device for the current treatment session reaches the target duration time for the treatment session, the acoustic output ends. Alternatively, according to a further set of examples, the generation of the acoustic output may continue in the event that the user ceases application of the skin treatment before the target duration time for the treatment session has been reached. It may be controlled to continue until the target duration time for the treatment session has been fully reached, or may be controlled to continue for a pre-determined period of time following cessation of application of treatment by the user, provided that the target duration time for the treatment session is not reached in the meantime. In these example implementations, the continuation of the acoustic output may prompt the user to continue with the skin treatment for a longer period of time.

In both sets of examples, a user interface may be provided which permits a user to manually suppress the generation of the acoustic output, i.e. the user is provided an option to force the sound to end (e.g. stop button in a connected app).

In some embodiments, the generating of the acoustic output comprises the processing device communicating with an audio file database storing a plurality of audio files, each encoding a respective acoustic output of a different respective time duration, and retrieving one or more of the audio files from the database. Within this set of embodiments, according to some examples, the processing device may be adapted to configure a duration of the acoustic output by selecting from the database an audio file encoding an acoustic output having a duration which substantially matches the target treatment session duration. According to other examples, the processing device may be adapted to configure a duration of the acoustic output by selecting from the database a plurality of audio files encoding acoustic outputs having durations which in combination substantially match the target treatment session duration. Alternatively, as noted above, a duration of the acoustic output may be configured on the fly, e.g. by using a MIDI file.

By way of example, one set of embodiments might be implemented as follows.

A user begins use of the device by triggering a start of a treatment session. This may be a manual operation by the user, for example wherein the user operates a user interaction element, such as a button, to initiate the start of a treatment session. For example, the user may operate a user interaction element which is used to trigger treatment events, as discussed above, and wherein this is interpreted by the processing device 32 as a trigger to initiate a start of a treatment session. By way of a further example, a software application installed on a mobile computing device associated with the skin treatment device 42 may permit a user to manually trigger start of a treatment session. By way of a further example, the skin treatment device 42 may comprise an integrated movements sensor for sensing movement of the device and wherein start of a treatment session is initiated when movement of the device is detected, or when movement of the device matching a certain pre-defined movement pattern is detected.

The start of the treatment session triggers the skin treatment subsystem 43 to begin operation. As discussed above, this could comprise automatic triggering of treatment events or could involve treatment events being triggered by a user operating a user control element.

Simultaneous with start of the treatment session, the processing device 32 begins control of the acoustic output device 44 to generate the acoustic output.

The acoustic output continues to play until the treatment session is detected to no longer be in progress, or until the target duration time of the treatment session has ended, whichever comes first. When the end of a treatment session is detected, the acoustic output may be terminated straight away. More preferably, when the end of a treatment session is detected, if the target duration time for the treatment session has not yet been reached, the acoustic output continuous to play for a pre-determined additional period of time before ending. This encourages the user to continue treatment. For example, the acoustic output may comprise a piece of music, a song, or a spoken text (e.g. a podcast). The user is encouraged by to continue use of the device until the natural end of the acoustic output.

End of a treatment session may be detected in different ways. One way is to detect cessation of operation of any one or more user control elements comprised by the device, indicating that the user has stopped use of the device. For example, it may be detected that no use has been made of one or more control elements within a pre-determined window of time. Additionally or alternatively, the device may comprise an integrated motion sensor, and end of a treatment session may be determined to have occurred when no movement of the device is detected for a pre-determined window of time.

As discussed above, one way of generating the acoustic output is to retrieve and play an audio file which encodes the acoustic output.

The audio file may be selected such that the total playing time of the acoustic output encoded on the audio file corresponds to an estimated time required for treatment of a particular target body part to be treated. As noted previously, the target body part to be treated may be indicated by the user, for example by means of input to the previously mentioned software application.

An initial estimate for the required playing time may be based on known average treatment times. For example, the processing device may be configured to query a treatment time database storing estimated treatment times for different body parts and to determine a target duration time for the treatment session as equal to the estimated treatment time for the target body part recorded in the treatment time database.

For future sessions, the required playing time may be updated based on actual usage or performance in a completed treatment session, for example based on an actual rate of triggering of treatment events (e.g. IPL pulses) by the user during a completed treatment session.

For example, the estimated treatment time for a given body part stored in the database may correspond to an estimated time to execute a pre-defined number of treatment events using the device. The processing device may be configured to update the estimated treatment time recorded in the treatment time database for the target body part in dependence upon an actual time taken by a user to perform the pre-defined number of treatment events during an actual treatment session.

To obtain an acoustic output of the required length from the audio files recorded in the audio file database, optionally a plurality of audio files may be stitched together which together make up the required time duration. In other words, in this case, the retrieving of an audio file from the audio file database may comprise the processing device communicating with an audio file database storing a plurality of audio files, each encoding a respective acoustic output of a different respective time duration, and retrieving a plurality of the audio files from the database. For example, the generated acoustic output may comprise two or more pieces of music, songs, or spoken texts in sequence in order to have the required total playing time.

The audio file database may be compiled from various different sources. The database could be at least partially compiled using accessible content on the internet. The database could be at least partially populated using audio files from a personal music library of the user. The database could be compiled using a dedicated library of audio files generated by the manufacturer. In some embodiments, a user subscription service may be provided wherein a user can subscribe for access to a library of audio files which may populate the audio file database.

In some embodiments, the audio file database may be populated with audio files having encoded acoustic outputs which have a psychological effect, e.g. for creating a pleasant user experience during treatment, e.g. music that might be used in a spa or a yoga class.

Music therapy has been used for both acute and chronic pain mitigation. One or more of the audio files might encode acoustic outputs which are songs designed to provide a music therapeutic effect.

In selecting the audio file to use to form the acoustic output, user preferences can be taken into account. The user may input preferences into a software application installed on a mobile computing device. These preferences may relate to semantic properties of the acoustic outputs encoded on the audio files, e.g. preferred music styles, desired atmosphere, or mood. The software application may present the user with a set of options with regards to different possible acoustic outputs, and wherein the user is permitted to select from among the set of options.

In addition to or instead of varying the maximum duration of the acoustic output in dependence upon the target characteristic of user operation of the device, according to some embodiments, the acoustic output may be generated so as to have a rhythm or beat frequency which approximately corresponds to a given rate which is dependent on a target characteristic of user operation. This is advantageous for example for skin treatment devices that operate by executing regular repeating sequences of skin treatment actions or events. For example, an IPL device applies energy to the skin in pulses. The user often moves such a device over the skin in a stepwise manner synchronous with the energy activations. The step-and-flash mode of an IPL device is a typical example. Alternatively, a user may need to move and activate a pulse of the device themselves at regular intervals. In either case, the rhythm of the acoustic output can be controlled to guide the user to make the necessary movements of the device or to trigger treatment events of the device with a frequency which matches a target frequency.

In this set of embodiments, the target characteristic of the user operation of the device includes a target movement pattern of the device by the user and/or a target activation frequency of the skin treatment sub-system by the user. The target movement pattern may include a target movement frequency. The at least one characteristic of the acoustic output which is configured by the processing device in dependence upon said target characteristic of the user operation of the device is a rhythm of the acoustic output. The processing device may be configured to generate the acoustic output such that the rhythm of the acoustic output corresponds rhythmically to the target movement pattern and/or target activation frequency.

The rhythm may have an associated rhythm frequency. The processing device may be configured to generate the acoustic output such that the rhythm frequency of the acoustic output matches the target movement frequency or activation frequency. In some embodiments, an audio file rhythm or frequency may be adjusted to match the target frequency of the characteristic of user operation of the device.

The target movement pattern may include or consist of a target rate or frequency of movement of the device by the user between different locations on the skin. It may additionally or alternatively comprise a target pattern of movement actions.

There are different ways to determine the target movement pattern and/or target activation frequency. In some examples, it may be pre-determined and stored as a data item in a memory or register. In further examples, in the case that the skin treatment device is operating in automatic triggering mode, in which treatment events, such as IPL pulses, are generated at regular intervals automatically, a target movement frequency may be set so as to match the automatic rate of triggering of the treatment events.

In the case that the skin treatment device is an IPL device, in some examples, the target movement frequency and/or target activation frequency may be set as equal to the maximum IPL pulse rate of the device. In this case, the total treatment time for a body part will be minimized. Alternatively, the target movement frequency and/or target activation frequency may be set at a rate lower than the maximum pulse rate. This lower rate may be perceived as more comfortable to the user. In this case, the experience will be made more enjoyable for the user.

In some embodiments, an AI algorithm may be used to automatically select content for the audio file that corresponds to a pre-determined rhythm. In other words, the retrieving the audio file comprises the processing device retrieving and applying an AI selection algorithm for selecting an audio file from the audio file database based on the target motion or activation frequency.

In addition, the selected content may be (slightly) modified to optimize the match to the pre-determined rhythm. For example, the processing device may be configured to retrieve an indication of a target movement pattern of the device and/ target activation frequency of the skin treatment sub-system by the user. The processing device may be configured to retrieve an audio file from the audio file database, the audio file having a rhythm frequency. The processing device may be configured to apply a modification to a pre-defined target movement frequency of the device and/ target activation frequency of the skin treatment sub-system by the user so as to improve a match between the rhythm frequency of the acoustic output encoded on the audio file and the target movement frequency of the device and/ target activation frequency of the skin treatment sub-system by the user.

Thus here, the target frequency of the characteristic of user operation of the device, e.g. a flash rate, is adjusted slightly to match the audio file rhythm or frequency.

New curated selections of acoustic outputs may be presented to the user shortly before it is time for the next treatment. This will encourage the user to plan and execute a session at the right time and thus adhere better to an optimal treatment schedule.

Where provided, the connected software application may be configured to keep track of a user treatment history, and curate acoustic outputs accordingly.

For example, the acoustic outputs may comprise a series of continued stories where the next part of the story is unlocked only after a specific number of days/weeks corresponding to the optimal treatment regime. Additionally, to be able to complete a full story, the user would need to adhere to the treatment regime for a certain period.

The content and the plot of the story can be adapted by the user behavior and decisions, for example with different possible endings being played conditionally upon the user's use of the device, e.g. for option A, the user must start treatment with the left leg first, for option B, the user must start treatment with the right leg first. This provides an interactive experience.

As noted previously, to adapt the acoustic output to the target characteristic of user operation, MIDI files can be used. For example, to match the tempo of the acoustic output to the IPL flash rate, MIDI files can be used since they allow for easily adapting the tempo to sync up with a desired rate. This way the user can follow a comfortable steady cadence preventing them from missing areas or overexposing certain areas.

Thus, according to some embodiments, the audio file is a MIDI audio file, and wherein the processing device is configured to control the acoustic output to have a rhythm frequency matching the target motion or activation frequency by modifying a rhythm frequency associated with the MIDI file. Thus here, the audio file rhythm or frequency may be adjusted to match the target frequency of the characteristic of user operation of the device.

Some concepts from music training could be also used to train a user on an optimal tempo. Some examples are as follows. A software application may be configured to guide the user to perform exercises using a metronome prior to a treatment session. This could help the user to adhere to a steady optimal tempo, helping the user to develop sense of steady rhythm. The tempo of the acoustic output could be configured to increase gradually to reach the maximum optimal speed, gently pushing the user towards the desired tempo. Concepts of music production such as using a clear beat, drums or bass sounds can help the user to reach the desired cadence. The acoustic output may be configured such that the music build-ups (increased music intensity) to encourage the user to follow the desired cadence, particularly toward the end of a treatment session.

According to one advantageous set of embodiments, the processing device may be configured to control the acoustic output device to generate an acoustic sound responsive to each movement of the device by the user and/or responsive to each activation of the skin treatment sub-system by the user; and wherein the acoustic sound is superposed atop the acoustic output associated with the audio file. This provides the user interactive guidance as to whether the target movement pattern or activation frequency is being followed.

By way of example, in this set of embodiments, the skin treatment device can effectively become a controller for the music which is played. In some examples, each button of the IPL device can be detected individually and, when triggered, may trigger reproduction of different sounds (e.g., kick drum, bass sound, high hat, etc.). Such sounds can be used as feedback as to usage of the device. For example, a good flashing rate may result in triggering a kick drum sound and less optimal flashing rate nay result in triggering a high hat. This way, the user receives feedback in real-time.

In some embodiments, to assist the user in becoming more intuitively trained as to the optimal flash rate, a MIDI file could be used which encodes a music piece in which one instrument is missing. This is often referred to as a "minus one" track or "karaoke" track. A user interface such as the aforementioned software application may permit the user to select an instrument which will be operated by the user control element(s) of the device, or which will be triggered when moving the device between positions on the skin. When the user follows the correct tempo or flash rate, the whole music piece will sound consonant and when the user misses the expected tempo, they will notice intuitively that the flashes are out of tempo.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A skin treatment system (30), comprising:
a user-operable skin treatment device (42) for application to skin, wherein the skin treatment device comprises a skin treatment sub-system (43) for administering a skin treatment to skin over the course of a treatment session, and wherein one or more characteristics of the skin treatment are controllable by the user through user operation of the device;
an acoustic output device (44) for generating an audible acoustic output;
a processing device (32) for controlling the acoustic output of the acoustic output device (44), the processing device (32) configured to:
obtain (12) an indication of a target characteristic (22) of the user operation of the device to be performed in a treatment session;
control (14) the acoustic output device (44) to generate an acoustic output,
wherein at least one characteristic of the acoustic output is configured in dependence upon the target characteristic of the user operation of the device.

2. The system of claim 1, wherein the at least one characteristic (22) of the acoustic output comprises at least one acoustic property of the acoustic output, optionally wherein the at least one acoustic property includes a duration of the acoustic output and/or a rhythm frequency of the acoustic output.

3. The system of claim 1 or 2, wherein
the target characteristic of the user operation of the device includes a target duration time of the treatment session;
the at least one characteristic of the acoustic output includes a duration time of the acoustic output; and
the acoustic output is controlled such that a duration time of the generated acoustic output is no longer than the target duration time for the treatment session.

4. The system of claim 3, wherein the obtaining an indication of the target duration time for the treatment session comprises:
obtaining an indication of a target body part to be treated; and
querying a treatment time database storing estimated treatment times for different body parts in accordance with the target body part to be treated.

5. The system of claim 4, wherein the estimated treatment time for a given body part corresponds to an estimated time to execute a pre-defined number of treatment events using the skin treatment subsystem, and wherein the processing device is further configured to update the estimated treatment time recorded in the treatment time database for the target body part in dependence upon an actual time taken by a user to perform the pre-defined number of treatment events during an actual treatment session.

6. The system of any of any preceding claim, wherein the processing device is configured to detect a start of a treatment session by:
detecting movement of the skin treatment device by a user using a motion sensor integrated in the skin treatment device;
detecting operation by the user of a user control element comprised by the skin treatment device, for example the pressing of a button by the user;
detecting a manual indication of an intended start of a treatment session by a user by means of a user interface operatively coupled with the processing device, for example by means of a software application installed on a mobile computing device which is operatively coupled with the processing device.

7. The system of any preceding claim,
wherein the target characteristic of the user operation of the device includes a target movement pattern of the device by the user and/or a target activation frequency of the skin treatment sub-system by the user;
wherein the processing device is configured to generate the acoustic output such that the rhythm of the acoustic output corresponds rhythmically to the target movement pattern and/or target activation frequency.

8. The system of any preceding claim,
wherein the generating of the acoustic output comprises retrieving one or more audio files from an audio file database and controlling the acoustic output device to generate an acoustic output based on the one or more audio files.

9. The system of claim 8, wherein the retrieving the audio file comprises:
communicating with an audio file database storing a plurality of audio files encoding different respective acoustic outputs differing from one another with regards to at least one acoustic property, e.g. duration and/or rhythm;
determining a target value of the at least one acoustic property for the acoustic output based on the target characteristic of user operation of the device; and
selecting one or more of the plurality of audio files in accordance with the target value of the at least one acoustic property.

10. The device of claim 9,
wherein the retrieving the audio file comprises retrieving an audio file which encodes an acoustic output with an associated rhythm which corresponds rhythmically to a target motion pattern and/or activation frequency of the device; and/or
wherein the retrieving the audio file comprises retrieving an audio file which encodes an acoustic output having a duration which matches or substantially matches a target duration time of the treatment session.

11. The device of claim 9 or 10, wherein the retrieving the audio file comprises the processing device retrieving and applying an AI selection algorithm for selecting the audio file based on the at least one acoustic property.

12. The device of any of claims 8-11,
wherein the retrieved audio file is a MIDI audio file, the MIDI audio file encoding an acoustic output having digitally encoded values for one or more acoustic properties;
wherein the processing device is configured to determine a target value of at least one acoustic property for the acoustic output based on the target characteristic of user operation of the device; and
wherein the processing device is configured to control the acoustic output to have the target value for the at least one acoustic property by modifying the digitally encoded value of said at least one acoustic property encoded in the MIDI file.

13. The skin treatment system of any preceding claim,
wherein the target characteristic of the user operation of the device includes a target movement pattern of the device by the user and/or a target activation frequency of the skin treatment sub-system by the user;
wherein the processing device is configured to control the acoustic output device to generate an acoustic sound responsive to each movement of the device by the user and/or responsive to each activation of the skin treatment sub-system by the user; and
wherein the acoustic sound is superposed atop the acoustic output associated with the audio file.

14. The skin treatment device of any preceding claim, wherein the skin treatment device is an Intense Pulsed Light, IPL, photo-epilation device.

15. A method (10) for providing interactive guidance to a user during a treatment session using a user-operable skin treatment device (42) to administer a skin treatment, wherein one or more characteristics of the skin treatment are controllable by the user through user operation of the device, wherein the method comprises:
obtaining (12) an indication of a target characteristic of the user operation of the device to be performed in a treatment session;
controlling (14) an acoustic output device to generate an acoustic output, wherein at least one characteristic of the acoustic output is configured in dependence upon the target characteristic of the user operation of the device.
